(19) Europäisches Patentamt
European Patent Office
Office européen des brevets

(11) Veröffentlichungsnummer: **0 141 045**
**A2**

(12) # EUROPÄISCHE PATENTANMELDUNG

(21) Anmeldenummer: **84108403.1**

(22) Anmeldetag: **17.07.84**

(51) Int. Cl.[4]: **C 07 D 417/14**
C 07 D 413/14, A 01 N 43/76
A 01 N 43/70

(30) Priorität: **21.10.83 DE 3338760**

(43) Veröffentlichungstag der Anmeldung:
**15.05.85 Patentblatt 85/20**

(84) Benannte Vertragsstaaten:
**BE CH DE FR GB IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65(DE)**

(72) Erfinder: **Krüger, Hans-Rudolf, Dr.**
**Kulmbacher Strasse 15**
**D-1000 Berlin 30(DE)**

(72) Erfinder: **Arndt, Friedrich, Dr.**
**Mühlenfeldstrasse 10**
**D-1000 Berlin 28(DE)**

(54) **2-Phenoxypropionsäurederivate von Pentiten vom Typ heterocyclischer Äther, Verfahren zur Herstellung dieser Verbindungen sowie diese enthaldende Mittel mit herbizider Wirkung.**

(57) Die Erfindung betrifft neue 2-Phenoxypropionsäurederivate von Pentiten vom Typ heterocyclischer Äther der allgemeinen Formel

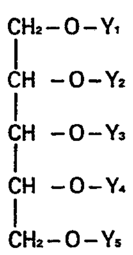

I

in der einer der Substituenten Y die Gruppe

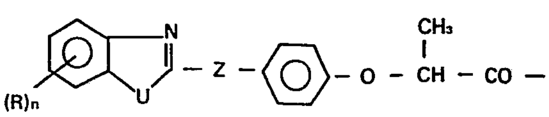

II

und die anderen vier Substituenten Y jeweils paarweise eine gegebenenfalls substituierte Methylengruppe der allgemeinen Formel

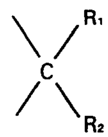

bedeuten, worin

$R_1$ und $R_2$ gleich oder verschieden sind und jeweils Wasserstoff, einen $C_1$-$C_{10}$-Alkylrest, einen ein- oder mehrfach durch Halogen, $C_1$-$C_6$-Alkoxy, Phenoxy, und/oder Halogenphenoxy substituierten $C_1$-$C_{10}$-Alkylrest, einen Aryl-$C_1$-$C_3$-alkylrest, einen ein- oder mehrfach durch $C_1$-$C_6$-Alkyl, Halogen, $C_1$-$C_6$/Alkoxy, Nitro und/oder Trifluormethyl substituierten Aryl-$C_1$-$C_3$-alkylrest, einen $C_3$-$C_8$-cycloaliphatischen Kohlenwasserstoffrest, einen aromatischen Kohlenwasserstoffrest oder einen ein- oder mehrfach durch $C_1$-$C_6$-Alkyl, Halogen, $C_1$-$C_6$-Alkoxy, Nitro und/oder Trifluormethyl substituierten aromatischen Kohlenwasserstoffrest oder $R_1$ und $R_2$ gemeinsam mit dem angrenzenden C-Atom einen $C_3$-$C_8$- cycloaliphatischen Kohlenwasserstoffrest darstellen,

R Halogen-, $C_1$-$C_6$-Alkoxy, $C_1$-$C_6$-Alkyl, Nitro und/oder die Trifluormethylgruppe,

U und Z jeweils ein Sauerstoff- oder ein Schwefelatom und n die Zahlen 0, 1 oder 2 bedeuten,

Verfahren zur Herstellung dieser Verbindungen sowie diese enthaltende herbizide Mittel.

EP 0 141 045 A2

Die Erfindung betrifft neue 2-Phenoxypropionsäurederivate von Pentiten vom Typ heterocyclischer Äther, Verfahren zur Herstellung dieser Verbindungen sowie diese enthaltende Mittel mit herbizider Wirkung.

2-Phenoxypropionsäurederivate von Pentiten vom Typ heterocyclischer Äther sind bisher nicht bekannt geworden.

Aufgabe der vorliegenden Erfindung ist die Schaffung neuer Wirkstoffe mit vorteilhafter herbizider Wirkung.

Diese Aufgabe wird erfindungsgemäß durch ein Mittel gelöst, das als neue Wirkstoffe mindestens ein 2-Phenoxypropionsäurederivat der allgemeinen Formel

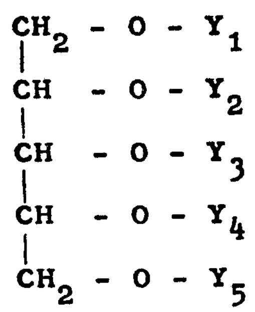

I

enthält,
in der einer der Substituenten Y die Gruppe

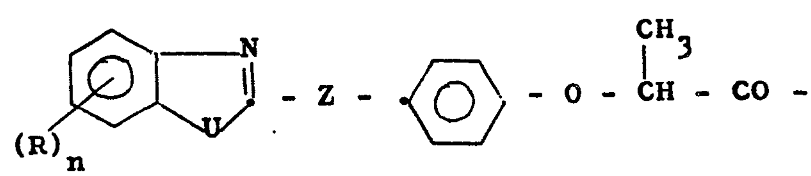

II

und die anderen vier Substituenten Y jeweils paarweise eine gegebenenfalls substituierte Methylengruppe der allgemeinen Formel

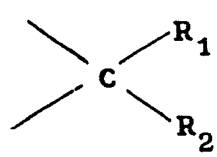

bedeuten, worin

-2-

$R_1$ und $R_2$ gleich oder verschieden sind und jeweils Wasserstoff, einen $C_1$-$C_{10}$-Alkylrest, einen ein- oder mehrfach durch Halogen, $C_1$-$C_6$-Alkoxy, Phenoxy und/oder Halogenphenoxy substituierten $C_1$-$C_{10}$-Alkylrest, einen Aryl-$C_1$-$C_3$-alkylrest, einen ein- oder mehrfach durch $C_1$-$C_6$-Alkyl, Halogen, $C_1$-$C_6$-Alkoxy, Nitro und/oder Trifluormethyl substituierten Aryl-$C_1$-$C_3$-alkylrest, einen $C_3$-$C_8$-cycloaliphatischen Kohlenwasserstoffrest, einen aromatischen Kohlenwasserstoffrest oder einen ein- oder mehrfach durch $C_1$-$C_6$-Alkyl, Halogen, $C_1$-$C_6$-Alkoxy, Nitro und/oder Trifluormethyl substituierten aromatischen Kohlenwasserstoffrest oder $R_1$ und $R_2$ gemeinsam mit dem angrenzenden C-Atom einen $C_3$-$C_8$-cycloaliphatischen Kohlenwasserstoffrest darstellen,
R Halogen-, $C_1$-$C_6$-Alkoxy, $C_1$-$C_6$-Alkyl, Nitro und/oder die Trifluormethylgruppe,
U und Z jeweils ein Sauerstoff- oder ein Schwefelatom und n die Zahlen 0, 1 oder 2 bedeuten.

Die erfindungsgemäßen Verbindungen treten als optische, gegebenenfalls auch als geometrische Isomere auf. Die einzelnen Isomeren und ihre Mischungen gehören auch zum Gegenstand der Erfindung.

Die erfindungsgemäßen Mittel eignen sich überraschenderweise zur Bekämpfung von Unkräutern sogar unter Schonung von Kulturpflanzen und bereichern daher den Stand der Technik auf diesem Gebiet erheblich.

Die erfindungsgemäßen Verbindungen sind im Vor- und Nachauflaufverfahren gegen ein breites Spektrum von Schadgräsern wirksam, gleichzeitig werden sie jedoch von zweikeimblättrigen Kulturpflanzen sowie einigen Getreidearten vertragen.

Die erfindungsgemäßen Mittel sind beispielsweise brauchbar zur selektiven Bekämpfung von schwer bekämpfbaren Ungräsern,



Postanschrift: Schering Aktiengesellschaft, Postfach 65 03 11, D-1000 Berlin 65 · Fur Besucher Berlin-Wedding, Mullerstraße 170-178 · Telegramme: Scheringchemie Berlin.
Vorstand Dr Herbert Asmis Dr. Christian Bruhn. Dr. Heinz Hannse. Horst Kramp. Dr. Klaus Pohle Dr Horst Witzel · Vorsitzender des Aufsichtsrats: Hans-Jürgen Hamann
Sitz der Gesellschaft Berlin und Bergkamen Handelsregister. AG Charlottenburg 93 HRB 283 und AG Kamen HRB 0061 · Berliner Commerzbank AG. Berlin. Konto-Nr 108700600 Bankleitzahl 100 400 00 Berliner Handels- und Frankfurter Bank. Berlin. Konto-Nr 70045224. Bankleitzahl 100 202 00 · Deutsche Bank Berlin AG. Konto-Nr 2415008. Bankleitzahl 100 700 00 Postscheckamt Berlin West Konto-Nr 11 75-101. Bankleitzahl 100 100 10

wie Avena fatua, Alopecurus myosuroides, Echinochloa crus galli, Setaria sp., Setaria faberi, Digitaria sanguinalis, Sorghum halepense, Poa annua, Bromus sp., Agropyron repens, Cynodon dactylon, Agrostis sp., Lolium sp., Eleusine indica, Rottboellia sp., Cenchrus sp., in Kulturen wie Zuckerrüben, Baumwolle, Soja, Erdnuß, Erbse, Sonnenblume, Raps und Kohlarten und in ausdauernden Kulturen, wie Obstanlagen, Reben und Plantagenkulturen.

Die erfindungsgemäßen Mittel können im Vorauflaufverfahren, vorzugsweise aber im Nachauflaufverfahren appliziert werden und besitzen vorteilhafterweise schon in niedrigen Aufwandmengen von 0,05 bis 5,0 kg Aktivsubstanz/ha eine gute Wirkung.

Die erfindungsgemäßen Verbindungen können entweder allein, in Mischung miteinander oder mit anderen Wirkstoffen angewendet werden. Gegebenenfalls können Entblätterungs-, Pflanzenschutz- oder Schädlingsbekämpfungsmittel je nach dem gewünschten Zweck zugesetzt werden.

Sofern eine Verbreiterung des Wirkungsspektrums beabsichtigt ist, können auch andere Biozide zugesetzt werden. Beispielsweise eignen sich als herbizid wirksame Mischungspartner diejenigen Wirkstoffe, die in Weed Abstracts, Vol. 31, No. 7, 1982, unter dem Titel "List of common names and abbreviations employed for currently used herbicides and plant growth regulators in Weed Abstracts" aufgeführt sind.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acyl-phosphatidylethanolaminen, Phosphatidylinosit, Phosphatidylserin, Lysolecithin und Phosphatidylglycerol.



Postanschrift: Schering Aktiengesellschaft, Postfach 65 03 11, D-1000 Berlin 65 · Für Besucher Berlin-Wedding, Müllerstraße 170-178 · Telegramme: Scheringchemie Berlin
Vorstand: Dr. Heribert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

Es wurde weiterhin gefunden, daßMittel enthaltend die erfindungsgemäßen Verbindungen in Mischung mit einigen bereits aus Weed Abstracts (l.c.) bekannten Herbiziden, wie Phenmedipham, Desmedipham und analogen Biscarbamaten, eine gesteigerte herbizide Wirkung entfalten.

Gegenstand der vorliegenden Erfindung sind daher außerdem herbizide Mischungen, die dadurch gekennzeichnet sind, daß sie mindestens eine Verbindung der allgemeinen Formel I in Mischung mit mindestens einer Verbindung der allgemeinen Formel

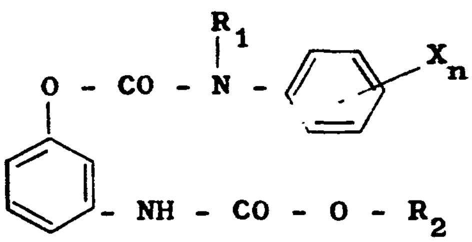

XI

enthalten, in der

$R_1$ Wasserstoff oder einen $C_1$-$C_3$-Alkylrest, $R_2$ einen $C_1$-$C_3$-Alkylrest, X einen $C_1$-$C_3$-Alkylrest und/oder Halogen und/oder einen CN-Rest und n 0, 1, 2 oder 3 bedeuten, in einem Gewichtsverhältnis von 1:10 bis 10:1, vorzugsweise von 1:3 bis 3:1.

Als besonders geeignete Mischungspartner der allgemeinen Formel XI sind zu nennen die an sich bekannten Verbindungen
Methyl-N-(3-(N'-(3'-methylphenyl)-carbamoyloxy)-phenyl)-carbamat, (Phenmedipham)
Äthyl-N-(3-(N'-phenylcarbamoyloxy)-phenyl)-carbamat, (Desmedipham)
und
Isopropyl-N-(3-(N'-äthyl-N'-phenylcarbamoyloxy)-phenyl-carbamat.

Als eine besonders geeignete Verbindung der allgemeinen Formel I kann
5-O-{2-[4-(6-Chlorbenzothiazol-2-yloxy)phenoxy]-propionyl}-1,2:3,4-bis-O-isopropylidenxylit



Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 · Berliner Commerzbank AG, Berlin, Konto-Nr. 108700600 Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008 Bankleitzahl 100 700 00 · Postscheckamt Berlin West, Konto-Nr. 1175-101 Bankleitzahl 100 100 10

als Mischungspartner eingesetzt werden.

Als weitere geeignete Biscarbamate, die in den erfindungsgemäßen Mischungen verwendet werden können, sind diejenigen zu benennen, die in den Deutschen Offenlegungsschriften Nr. 84 21 584, 15 67 151 und 24 13 933 aufgeführt sind.

Die herbizide Wirkung dieser Biscarbamate ist bereits bekannt, doch zeigt sich diese unter besonderen Bedingungen noch verbesserungsbedürftig, zum Beispiel bei der Bekämpfung von sehr resistenten dikotylen Unkräutern und Ungräsern insbesondere in Kulturen von Zuckerrüben und Futterrüben.

Überraschenderweise zeigen die erfindungsgemäßen Mischungen eine erheblich gesteigerte Wirkung gegen derartige Unkräuter und Ungräser, ohne daß die hiermit behandelten Rübenpflanzen geschädigt wurden.

Es handelt sich also um eine vorteilhafte Veränderung der Herbizidwirkung der genannten Biscarbamate, indem diese selbst bei erschwerten Bedingungen, wie sehr hohen Aufwandmengen, nicht auf Rüben schädigend wirken bei gleichzeitiger Erweiterung ihres Wirkungsspektrums auf Ungräser.

Das Gewichtsverhältnis der Komponenten in den erfindungsgemäßen Mischungen soll etwa von 1:10 bis 10:1, vorzugsweise von 1:3 bis 3:1, betragen.

Die bevorzugten Wirkstoffmengen zur selektiven Unkrautbekämpfung bewegen sich von etwa 0,5 bis 5,0 kg/ha für die Verbindungen der Biscarbamate und von etwa 0,05 bis 5,0 kg/ha für die Verbindungen der heterocyclischen Phenyläther.

Von Vorteil ist außerdem der Zusatz eines geeigneten grenzflächenaktiven Stoffes zwischen 0,25 bis5,0 kg/ha und anderen Wirkungsverstärkern wie zum Beispiel nicht phytotoxische Öle.

-6-

**Postanschrift: Schering Aktiengesellschaft, Postfach 65 03 11, D-1000 Berlin 65** · Fur Besucher Berlin-Wedding Mullerstraße 170-178 · Telegramme: Scheringchemie Berlin

Vorstand: Dr Herbert Asmis Dr Christian Bruhn Dr Heinz Hannse Horst Kramp Dr Klaus Pohle Dr Horst Witzel Vorsitzender des Aufsichtsrats: Hans-Jurgen Hamann
Sitz der Gesellschaft Berlin und Bergkamen Handelsregister AG Charlottenburg 93 HRB 283 und AG Kamen HRB 0061 Berliner Commerzbank AG, Berlin Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG Konto-Nr 2415008, Bankleitzahl 100 700 00 Postscheckamt Berlin West Konto-Nr 11 75 101 Bankleitzahl 100 100 10

Von den erfindungsgemäßen Verbindungen zeichnen sich durch die beschriebenen Wirkungen insbesondere diejenigen aus, bei denen in der oben angeführten allgemeinen Formel I $R_1$ und $R_2$ gleich oder verschieden sind und jeweils Wasserstoff, Methyl, Äthyl, Propyl, Isopropyl, n-Butyl, sec-Butyl, tert.-Butyl, 2,2-Dimethyl-1-propyl, n-Pentyl, n-Heptyl, n-Octyl, n-Decyl, Chlormethyl, Brommethyl, Fluormethyl, Dichlormethyl, Trifluormethyl, Methoxymethyl, Äthoxymethyl, Phenoxymethyl, 4-Chlorphenoxymethyl, Chloräthyl, Bromäthyl, 2-Äthoxyäthyl, 2-Phenoxyäthyl, Cyclopropyl, Cyclopentyl, Cyclohexyl, Benzyl, 2-Phenyläthyl, Phenyl, 2-Chlorphenyl, 3-Chlorphenyl, 4-Chlorphenyl, 3,4-Dichlorphenyl, 4-Methoxyphenyl, 4-Nitrophenyl, 2,4-Dichlorphenyl bedeuten oder $R_1$ und $R_2$ gemeinsam unter Ausbildung einer Spiroverbindung eine Polmethylengruppe $-(CH_2)_m-$ darstellen, wobei m die Zahl 4 oder 5 darstellt.

Unter den mit

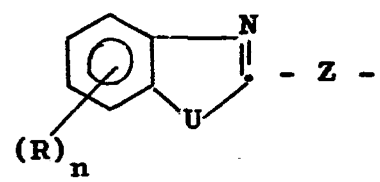

bezeichneten Resten ist vorzugsweise der 6-Chlor-2-benzthiazolyloxy-und der 6-Chlor-2-benzoxazolyloxy-rest zu verstehen.

Bevorzugte Pentitderivate der allgemeinen Formel I sind überdies solche, in denen

1) $Y_1$ die Gruppe

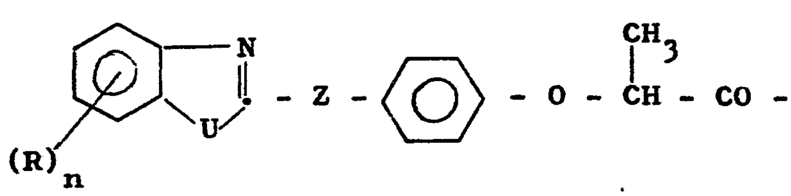 II

und $Y_2$ und $Y_3$ zusammen und $Y_4$ und $Y_5$ zusammen jeweils eine gegebenenfalls substituierte Methylengruppe

-7-

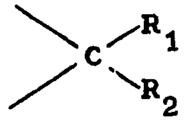

darstellen und

2) $Y_3$ die Gruppe der allgemeinen Formel II und $Y_1$ und $Y_2$ zusammen und $Y_4$ und $Y_5$ zusammen jeweils eine gegebenenfalls substituierte Methylengruppe und

3) $Y_5$ die Gruppe der allgemeinen Formel II und $Y_1$ und $Y_2$ zusammen und $Y_3$ und $Y_4$ zusammen jeweils eine gegebenenfalls substituierte Methylengruppe bedeuten.

Zweckmäßig werden die erfindungsgemäßen Wirkstoffe oder deren Mischung in Form von Zubereitungen wie Pulvern, Streumitteln, Granulaten, Lösungen, Emulsionen oder Suspensionen unter Zusatz von flüssigen und/oder festen Trägerstoffen beziehungsweise Verdünnungsmitteln und gegebenenfalls von Netz-, Haft-, Emulgier- und/oder Dispergierhilfsmitteln, angewandt, vorzugsweise in Form von Emulsionskonzentraten.

Geeignete flüssige Trägerstoffe sind zum Beispiel Wasser, aliphatische und aromatische Kohlenwasserstoffe, wie Benzol, Toluol, Xylol, Cyclohexanon, Isophoron, Dimethylsulfoxid, Dimethylformamid, weiterhin Mineralölfraktionen.

Als feste Trägerstoffe eignen sich Mineralerden, zum Beispiel Tonsil, Silicagel, Talkum, Kaolin, Attaclay, Kalkstein, Kieselsäure und pflanzliche Produkte, zum Beispiel Mehle.

An oberflächenaktiven Stoffen sind zu nennen zum Beispiel Calciumligninsulfonat, Polyoxyäthylen-alkylphenoläther, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

-8-

**Postanschrift: Schering Aktiengesellschaft, Postfach 65 03 11, D-1000 Berlin 65** · Fur Besucher Berlin-Wedding, Mullerstraße 170-178 Telegramme Scheringchemie Berlin
Vorstand Dr Herbert Asmis Dr Christian Bruhn Dr Heinz Hannse Horst Kramp Dr Klaus Pohle Dr Horst Witzel Vorsitzender des Aufsichtsrats Hans-Jurgen Hamann Sitz der Gesellschaft Berlin und Bergkamen Handelsregister AG Charlottenburg 93 HRB 283 und AG Kamen HRB 0061 Berliner Commerzbank AG Berlin Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG, Konto-Nr 2415008 Bankleitzahl 100 700 00 Postscheckamt Berlin West Konto-Nr 1175-101 Bankleitzahl 100 100 10

Der Anteil des beziehungsweise der Wirkstoffe(s) in den verschiedenen Zubereitungen kann in weiten Grenzen variieren. Beispielsweise enthalten die Mittel etwa 5 bis 95 Gewichtsprozente Wirkstoffe, etwa 95 bis 5 Gewichtsprozente flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen von etwa 100 bis 1000 Liter /ha. Eine Anwendung der Mittel im sogenannten Low-Volume- und Ultra-Low-Volume-Verfahren ist ebenso möglich wie ihre Applikation in Form von sogenannten Mikrogranulaten.

Zur Herstellung der Zubereitungen werden zum Beispiel die folgenden Bestandteile eingesetzt:

A. Spritzpulver

a) 40 Gewichtsprozent Wirkstoff
25 Gewichtsprozent Tonmineralien
20 Gewichtsprozent kolloidale Kieselsäure
10 Gewichtsprozent Zellpech
5 Gewichtsprozent oberflächenaktive Stoffe auf der Basis einer Mischung des Calciumsalzes der Ligninsulfonsäure mit Alkylphenolpolyglycoläthern

b) 25 Gewichtsprozent Wirkstoff
60 Gewichtsprozent Kaolin
10 Gewcihtsprozent kolloidale Kieselsäure
5 Gewcihtsprozent oberflächenaktive Stoffe auf Basis des Natriumsalzes des N-Methyl-N-oleyl-taurins und des Calciumsalzes der Ligninsulfonsäure



Postanschrift: Schering Aktiengesellschaft, Postfach 65 03 11, D-1000 Berlin 65 · Fur Besucher Berlin-Wedding, Mullerstraße 170-178 Telegramme Scheringchemie E
Vorstand: Dr. Herbert Asmis Dr. Christian Brunn Dr. Heinz Hannse, Horst Kramp, Dr. Klaus Pohle, Dr. Horst Witzel · Vorsitzender des Aufsichtsrats: Hans-Jurgen Har
Sitz der Gesellschaft: Berlin und Bergkamen Handelsregister: AG Charlottenburg 93 HRB 283 und AG Kamen HRB 0061 Berliner Commerzbank AG Berlin Kont
108700600 Bankleitzahl 100 400 00 Berliner Handels- und Frankfurter Bank Berlin, Konto-Nr 70045224, Bankleitzahl 100 202 00 Deutsche Bank Berlin AG, Kont
2415008 Bankleitzahl 100 700 00 Postscheckamt Berlin West, Konto-Nr 1175-101, Bankleitzahl 100 100 10

c) 10 Gewichtsprozent Wirkstoff
60 Gewichtsprozent Tonmineralien
15 Gewichtsprozent kolloidale Kieselsäure
10 Gewichtsprozent Zellpech
5 Gewichtsprozent oberflächenaktive Stoffe auf Basis des Natriumsalzes des N-Methyl-N-oleyl-taurins und des Calciumsalzes der Ligninsulfonsäure

B. Paste

45 Gewichtsprozent Wirkstoff
5 Gewichtsprozent Natriumaluminiumsilikat
15 Gewichtsprozent Cetylpolyglykoläther mit 8 Mol Äthylenoxid
2 Gewichtsprozent Spindelöl
10 Gewichtsprozent Polyäthylenglykol
23 Teile Wasser

C. Emulsionskonzentrat

a) 25 Gewichtsprozent Wirkstoff
15 Gewichtsprozent Cyclohexanon
55 Gewichtsprozent Xylol
5 Gewichtsprozent Mischung von Nonylphenylpolyoxyäthylen oder Calciumdodecylbenzolsulfonat

b) 10 Gewichtsprozent Wirkstoff
6 Gewichtsprozent Cyclohexanon
36 Gewichtsprozent Xylol
12 Gewichtsprozent Mischung von Nonylphenylpolyoxyäthylen oder Calciumdodecylbenzolsulfonat
36 Gewichtsprozent Mineralöl mit hohem Paraffingehalt

-10-

Die neuen erfindungsgemäßen Verbindungen lassen sich zum **Beispiel herstellen, indem man**

A) Verbindungen der allgemeinen Formel

$$\begin{array}{l} CH_2 - O - Y'_1 \\ CH - O - Y'_2 \\ CH - O - Y'_3 \\ CH - O - Y'_4 \\ CH_2 - O - Y'_5 \end{array} \qquad III$$

in der einer der Substituenten Y' Wasserstoff und die anderen vier Substituenten Y' jeweils paarweise eine gegebenenfalls substituierte Methylengruppe

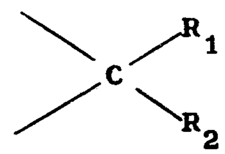

bedeuten mit Verbindungen der allgemeinen Formel

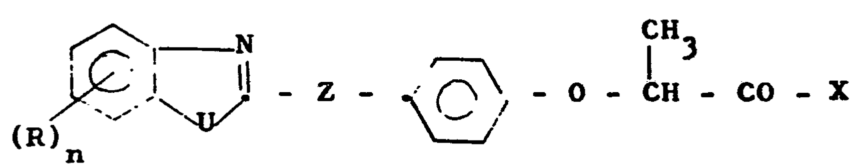

IV

gegebenenfalls in Gegenwart von säurebindenden Mitteln und/oder eines Katalysators reagieren läßt oder

B) Verbindungen der allgemeinen Formel

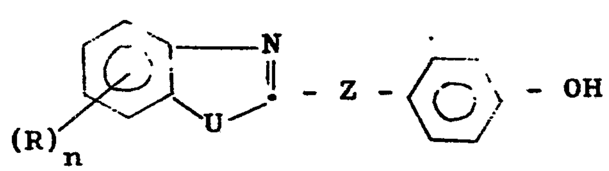

V

mit einem α-Halogenpropionsäurepentitester der allgemeinen Formel



Postanschrift: Schering Aktiengesellschaft, Postfach 65 03 11, D-1000 Berlin 65 · Für Besucher Berlin-Wedding, Müllerstraße 170-178 Telegramme Scheringchemie Berlin
Vorstand Dr. Herbert Asmis Dr. Christian Bruhn, Dr. Heinz Hannse, Horst Kramp, Dr. Klaus Pohle, Dr. Horst Witzel · Vorsitzender des Aufsichtsrats Hans-Jürgen Haman
Sitz der Gesellschaft Berlin und Bergkamen Handelsregister AG Charlottenburg 93 HRB 283 und AG Kamen HRB 0061 Berliner Commerzbank AG, Berlin, Konto-Nr. 108700600 Bankleitzahl 100 400 00 Berliner Handels- und Frankfurter Bank Berlin, Konto-Nr 70045224, Bankleitzahl 100 202 00 Deutsche Bank Berlin AG, Konto-Nr. 2415008 Bankleitzahl 100 700 00 Postscheckamt Berlin West Konto-Nr 1175-101, Bankleitzahl 100 100 10

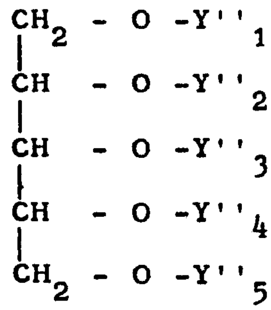

VI

in der einer der Substituenten Y'' die Gruppe

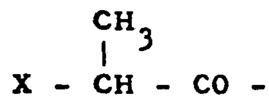

VII

und die anderen vier Substituenten Y'' jeweils paarweise eine gegebenenfalls substituierte Methylengruppe

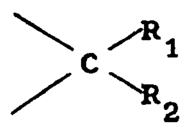

bedeuten, gegebenenfalls in Gegenwart von säurebindenden Mitteln und/oder eines Katalysators reagieren läßt oder, im Fall, daß Z allein Sauerstoff bedeutet,

C) Verbindungen der allgemeinen Formel

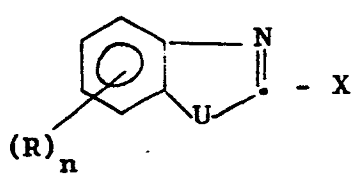

VIII

mit einem Hydrochinon - Milchsäureäther - derivat der allgemeinen Formel

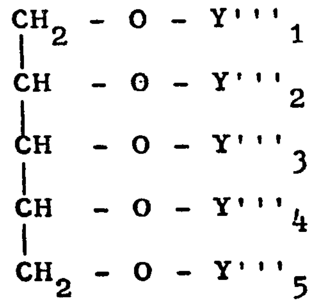

IX

-12-

in der eine der Substituenten Y''' die Gruppe

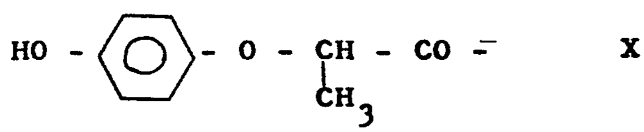

und die anderen vier Substituenten Y''' jeweils paarweise eine gegebenenfalls substituierte Methylengruppe der allgemeinen Formel

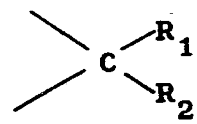

bedeuten, gegebenenfalls in Anwesenheit von säurebindenden Mitteln und/oder eines Katalysators reagieren läßt, worin R, $R_1$, $R_2$, n und Z die oben genannte Bedeutung haben und X ein Halogenatom, vorzugsweise Chlor oder Brom, darstellen.

Die Umsetzung der Reaktionspartner erfolgt zwischen -10 und 150°C, im allgemeinen jedoch zwischen Raumtemperatur und Rückflußtemperatur des entsprechenden Reaktionsgemisches.

Die Reaktionsdauer beträgt 1 bis 72 Stunden. In der Regel erfolgen die Reaktionen bei Normaldruck oder leichtem Überdruck. Zur Synthese der erfindungsgemäßen Verbindungen werden die Reaktanden in etwa äquimolaren Mengen eingesetzt. Geeignete Reaktionsmedien sind gegenüber den Reaktanden inerte Lösungsmittel. Die Wahl der Lösungs- beziehungsweise Suspensionsmittel richtet sich nach dem Einsatz der entsprechenden Alkyl- beziehungsweise Acylhalogenide und der angewandten Säureakzeptoren. Als Lösungs- beziehungsweise Suspensionsmittel seien beispielsweise genannt aliphatische und aromatische Kohlenwasserstoffe, wie Petroläther, Cyclohexan, Hexan, Heptan, Benzol, Toluol, Xylole; halogenierte Kohlenwasserstoffe, wie Methylenchlorid, Äthylenchlorid, Chlorbenzol, Chloroform, Tetrachlorkohlen-



Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 Berliner Commerzbank AG Berlin, Konto-Nr. 108700600 Bankleitzahl 100 400 00 Berliner Handels- und Frankfurter Bank Berlin Konto-Nr. 70045224 Bankleitzahl 100 202 00 Deutsche Bank Berlin AG, Konto-Nr. 2415008 Bankleitzahl 100 700 00 Postscheckamt Berlin West Konto-Nr. 1175-101, Bankleitzahl 100 100 10

stoff, Tetrachloräthylen; Äther, wie Diathyläther, Diisopropyläther, Anisol, Dioxan, Tetrahydrofuran; Carbonsäurenitrile, wie Acetonitril, Propionitril; Carbonsäureamide, wie Dimethylformamid; Dimethylsulfoxid; Ketone, wie Aceton, Diäthylketon, Methyläthylketon; Alkohole, wie Methanol, Äthanol, Propanol, Butanol und Gemischen solcher Lösungsmittel untereinander. In einigen Fällen kann auch der Reaktionspartner selbst als Lösungsmittel dienen.

Als Säureakzeptoren eignen sich organische Basen, wie zum Beispiel Triäthylamin, Trimethylamin, N,N-Dimethylanilin, Pyridin und Pyridinbasen (4-Dimethylaminopyridin) oder anorganische Basen wie Oxide, Hydroxide, Carbonate, Hydrogencarbonate und Alkoholate von Alkali- und Erdalkalimetallen sowie Alkalisalze von Carbonsäuren (KOH, NaOH, $Na_2CO_3$, $CH_3COONa$). Flüssige Basen wie Pyridin können gleichzeitig als Lösungsmittel eingesetzt werden. Entstehender Halogenwasserstoff kann in manchen Fällen auch mittels Durchleiten von Inertgas zum Beispiel Stickstoff, aus dem Reaktionsgemisch entfernt werden oder an Molekularsieb adsorbiert werden.

Die Gegenwart eines Reaktionskatalysators kann von Vorteil sein. Als Katalysatoren sind Kaliumjodid und Oniumverbindungen geeignet, wie quaternäre Ammonium-, Phosphonium- und Arsoniumverbindungen sowie Sulfoniumverbindungen. Ebenfalls geeignet sind Polyglykoläther, insbesondere cyclische, wie zum Beispiel 18-Krone-6 und tertiäre Amine, wie zum Beispiel Tributylamin. Bevorzugte Verbindungen sind quaternäre Ammoniumverbindungen, wie zum Beispiel Benzyltriäthylammoniumchlorid und Tetrabutylammoniumbromid.

Die nach oben genannten Verfahren hergestellten erfindungsgemäßen Verbindungen können nach den üblichen Verfahren aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion.

-14-

**Postanschrift: Schering Aktiengesellschaft, Postfach 65 03 11, D-1000 Berlin 65** · Für Besucher: Berlin-Wedding Müllerstraße 170-178 Telegramme: Scheringchemie Berlin
Vorstand: Dr. Herbert Asmis Dr. Christian Bruhn Dr. Heinz Hannse Horst Kramp Dr. Klaus Pohle Dr. Horst Witzel Vorsitzender des Aufsichtsrats: Hans-Jürgen Hamann Sitz der Gesellschaft: Berlin und Bergkamen Handelsregister: AG Charlottenburg 93 HRB 283 und AG Kamen HRB 0061 Berliner Commerzbank AG Berlin Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG Konto-Nr 2415008 Bankleitzahl 100 700 00 Postscheckamt Berlin West Konto-Nr 1175 101 Bankleitzahl 100 100 10

Ein erhöhter Reinheitsgrad kann in der Regel durch säulenchromatographische Aufreinigung sowie durch fraktionierte Destillation erhalten werden.

Die erfindungsgemäßen Verbindungen stellen in der Regel fast farb- und geruchlose Flüssigkeiten dar, aber auch zum Teil Kristalle, die schwerlöslich in Wasser, bedingt löslich in aliphatischen Kohlenwasserstoffen wie Petroläther, Hexan, Pentan und Cyclohexan; gut löslich in halogenierten Kohlenwasserstoffen wie Chloroform, Methylenchlorid und Tetrachlorkohlenstoff, aromatischen Kohlenwasserstoffen wie Benzol, Toluol und Xylol, Äther wie Diäthyläther, Tetrahydrofuran und Dioxan, Carbonsäurenitrilen wie Acetonitril, Ketonen wie Aceton, Alkoholen wie Methanol und Äthanol, Carbonsäureamiden wie Dimethylformamid und Sulfoxide wie Dimethylsulfoxid sind.

Die Verbindungen der allgemeinen Formel I besitzen stets in der Gruppe

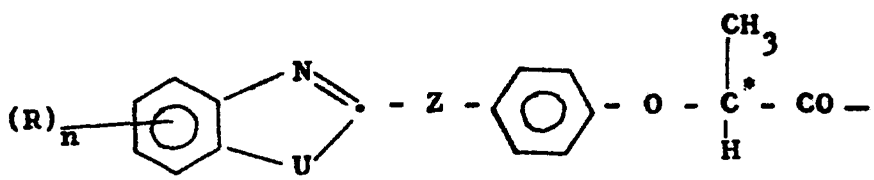

II

ein asymmetrisches Kohlenstoffatom C*. Bei der Synthese fallen die Wirkstoffe normalerweise als racemisches Gemisch an und können in bekannter Weise, beispielsweise über die entsprechenden Diastereomeren anhand bekannter Kristallisationsverfahren wie die optischen Antipoden gespalten werden.

Die Synthese einer optisch reinen Verbindung der Formel I ist beipielsweise möglich, wenn man bei deren Herstellung von einer optisch eindeutigen 2-Halogenpropionsäure ausgeht. Die optischen Antipoden von I besitzen unterschiedliche biologische Aktivität; in der Regel ist die D-Form herbizid wirksamer.

-15-

Die Ausgangsverbindungen zur Herstellung der erfindungsgemäßen Verbindungen sind an sich bekannt oder können nach an sich bekannten Verfahren hergestellt werden.

Die folgenden Beispiele erläutern die Herstellung der erfindungsgemäßen Pentitester.

-16-

Postanschrift: Schering Aktiengesellschaft, Postfach 65 03 11, D-1000 Berlin 65 · Fur Besucher Berlin-Wedding, Mullerstraße 170-178 · Telegramme Scheringchemie Berlin
Vorstand Dr Herbert Asmis Dr Christian Bruhn Dr Heinz Hannse Horst Kramp Dr Klaus Pohle Dr Horst Witzel Vorsitzender des Aufsichtsrats Hans-Jurgen Hamann Sitz der Gesellschaft Berlin und Bergkamen Handelsregister AG Charlottenburg 93 HRB 283 und AG Kamen HRB 0061 Berliner Commerzbank AG Berlin Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG Konto-Nr 2415008 Bankleitzahl 100 700 00 Postscheckamt Berlin West Konto-Nr 1175-101 Bankleitzahl 100 100 10

BEISPIEL 1

5-O-{2-/4-(6-Chlorbenzothiazol-2-yloxy)phenoxy7-propionyl}-1,2:3,4-bis-O-isopropylidenxylit

4,88 g (0,021 Mol) 1,2:3,4-Bis-O-(isopropyliden)-xylit werden in 60 ml Methylenchlorid vorgelegt und bei 5°C mit 4,23 g (0,042 Mol) Triäthylamin versetzt.

Anschließend tropft man hierzu eine Lösung von 7,75 g (0,021 Mol) 2-/4-(6-Chlorbenzothiazol-2-yloxy)-phenoxy7-propionsäurechlorid in 40 ml Methylenchlorid unter Eiskühlung bei 5°C. Es wird noch 3 Stunden bei Raumtemperatur nachgerührt. Das Reaktionsgemisch wird zur Trockne eingeengt und anschließend der Rückstand mit 300 ml Essigester aufgenommen. Die erst mit Wasser, anschließend mit gesättigter Kaliumcarbonatlösung gewaschenen Essigesterextrakte werden über Magnesiumsulfat getrocknet und anschließend filtriert. Nach Abziehen des Lösungsmittels wird das zurückbleibende Öl säulenchromatographisch (Mitteldruck) an Kieselgel (Merck 0,02-0,06mm; Eluens: Hexan/Essigester 9:1) aufgereinigt.

Ausbeute: 7,2 g = 60,8 % der Theorie
$n_D^{20}$: 1,5471
DC: Laufmittel = $CH_2Cl_2/CH_3OH$ (95:5)
$R_f$-Wert: 0,77

| | | | | | |
|---|---|---|---|---|---|
| Analyse: | Berechnet | C 57,49% | H 5,36% | N 2,48% | Cl 6,28% |
| | Gefunden | C 57,22% | H 5,61% | N 2,43% | Cl 6,27% |



Postanschrift Schering Aktiengesellschaft, Postfach 65 03 11, D-1000 Berlin 65 · Fur Besucher Berlin-Wedding, Mullerstraße 170-178 Telegramme Scheringchemie Berlin
Vorstand: Dr. Heinz Asmis, Dr. Christian Bruhn, Dr. Heinz Hannse, Horst Kramp, Dr. Klaus Pohle, Dr. Horst Witzel Vorsitzender des Aufsichtsrats Hans-Jurgen Hamann Sitz der Gesellschaft Berlin und Bergkamen Handelsregister AG Charlottenburg 93 HRB 283 und AG Kamen HRB 0061 Berliner Commerzbank AG, Berlin Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG Konto-Nr 2415008 Bankleitzahl 100 700 00 Postscheckamt Berlin West Konto-Nr 1175-101 Bankleitzahl 100 100 10

BEISPIEL 2

5-O-{2-[4-(6-Chlorbenzoxazol-2-yloxy)-phenoxy]-propionyl}-1,2:3,4-bis-O-isopropylidenxylit

5,0 g (0.019 Mol) 4-(6-Chlorbenzoxazol-2-yloxy)-phenol werden zu einer Suspension von 2,9 g (0,021 Mol) Kaliumcarbonat in 100 ml wasserfreiem Acetonitril gegeben.

Anschließend fügt man hierzu bei Raumtemperatur 7,71 g (0,021 Mol) 2-Brompropionyl-1,2:3,4-bis-O-isopropylidenxylit und erwärmt anschließend 4 Stunden auf 80°C. Dann wird das Reaktionsgemisch bei 40°C unter Wasserstrahlvakuum eingeengt. Der verbleibende Rückstand wird auf Wasser gegeben und dreimal mit je 100 ml Essigester extrahiert. Die über Magnesiumsulfat getrockneten Extrakte werden filtriert und unter Vakuum eingeengt. Das verbleibende Öl wird säulenchromatographisch (Mitteldruck) an Kieselgel (Merck 0,02-0,06 mm; Eluens: Hexan/Essigester 9:1) aufgereinigt.

| | |
|---|---|
| Ausbeute: | 5,25 g = 50,1 % der Theorie |
| $n_D^{20}$: | 1,5331 |
| DC: | Laufmittel = Hexan/Essigester (1:1) |
| | $R_f$-Wert: 0,60 |

| | | | | | |
|---|---|---|---|---|---|
| Analyse: | Berechnet | C 59,17 % | H 5,51 % | N 2,55 % | Cl 6,47 % |
| | Gefunden | C 58,96 % | H 5,68 % | N 2,37 % | Cl 6,30 % |

In analoger Weise lassen sich die weiteren erfindungsgemäßen Verbindungen herstellen.



Postanschrift: Schering Aktiengesellschaft, Postfach 65 03 11, D-1000 Berlin 65 · Für Besucher Berlin-Wedding, Müllerstraße 170-178 Telegramme Scheringchemie Berlin
Vorstand Dr Herbert Asmis Dr Christian Brunn Dr Heinz Hannse Horst Kramp Dr Klaus Pohle Dr Horst Witzel · Vorsitzender des Aufsichtsrats Hans-Jürgen Hamann Sitz der Gesellschaft Berlin und Bergkamen Handelsregister AG Charlottenburg 93 HRB 283 und AG Kamen HRB 0061 · Berliner Commerzbank AG Berlin Konto-Nr 108700600 Bankleitzahl 100 400 00 Berliner Handels- und Frankfurter Bank Berlin Konto-Nr 70045224 Bankleitzahl 100 202 00 · Deutsche Bank Berlin AG Konto-Nr 2415008 Bankleitzahl 100 700 00 Postscheckamt Berlin West Konto-Nr 1175-101 Bankleitzahl 100 100 10

| Beispiel Nr. | Name der Verbindungen | Physikalische Konstante |
|---|---|---|
| 3 | 5-O-{2-[4-(Benzothiazol-2-yloxy)-phenoxy]-propionyl}-1,2:3,4-bis-O-isopropylidenxylit | $n_D$20: 1,5471 |
| 4 | 5-O-{2-[4-(6-chlorbenzothiazol-2--yloxy)-phenoxy]-propionyl}-1,2:3,4--bis-O-(ethylpropyliden)-xylit | $n_D$20: 1,5451 |
| 5 | 5-O-{2-[4-(6-Chlorbenzothiazol-2--yloxy)-phenoxy]-propionyl}-1,2:3,4-bis-O-(1-methylpropyliden)-xylit | $n_D$20: 1,5381 |
| 6 | 5-O-{2-[4-(6-Chlorbenzothiazol-2--yloxy)-phenoxy]-propionyl}-1,2:3,4-bis-O-ethyliden-xylit | Fp.: 65-67°C |
| 7 | 5-O-{2-[4-(6-Chlorbenzothiazol-2--yloxy)-phenyloxy]-propionyl}--1,2:3,4-bis-O-(methyl-octyl-methylen)-xylit | $n_D$20: 1,5266 |
| 8 | 5-O-{2-[4-(6-Chlorbenzothiazol--2-yloxy)-phenoxy]-propionyl}-1,2:3,4-bis-O-(4-chlorphenyl-methylen)-xylit | zähes Öl |
| 9 | 5-O-{2-[4-(Benzoxazol-2-yloxy)-phenoxy]-propionyl}-1,2:3,4-bis-O-isopropylidenxylit | $n_D$20: 1,5293 |
| 10 | 5-O-{2-[4-(6-Chlorbenzoxazol-2--yloxy)-phenoxy]-propionyl}-1,2:3,4-bis-O-(ethylpropyliden)-xylit | $n_D^{20}$: 1,5304 |
| 11 | 5-O-{2-[4-(6-Chlorbenzoxazol-2--yloxy)-phenoxy]-propionyl}-1,2:3,4-bis-O-(1-methylpropyliden)--xylit | $n_D^{20}$: 1,5322 |
| 12 | 5-O-{2-[4-(6-Chlorbenzoxazol-2--yloxy)-phenoxy]-propionyl}-1,2:3,4-bis-O-ethyliden-xylit | Fp.: 127-128°C |
| 13 | 5-O-{2-[4-(6-Chlorbenzoxazol-2--yloxy)-phenoxy]-propionyl}-1,2:3,4-bis-O-(methyl-octylmethylen)-xylit | zähes Öl |
| 14 | 5-O-{2-[4-(6-Chlorbenzoxazol-2--yloxy)-phenoxy]-propionyl}-1,2:3,4-bis-O-(4-chlorphenylmethylen-)-ylit | zähes Öl |

-19-

Postanschrift: Schering Aktiengesellschaft, Postfach 65 03 11, D-1000 Berlin 65 · Für Besucher Berlin-Wedding Müllerstraße 170-178 Telegramme Scheringchemie Berlin

Vorstand: Dr. Herbert Asmis Dr. Christian Bruhn Dr. Heinz Hannse Horst Kramp Dr. Klaus Pohle Dr. Horst Witzel Vorsitzender des Aufsichtsrats: Hans-Jürgen Hamann Sitz der Gesellschaft: Berlin und Bergkamen Handelsregister: AG Charlottenburg 93 HRB 283 und AG Kamen HRB 0061 Berliner Commerzbank AG Berlin Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG Konto-Nr 2415008 Bankleitzahl 100 700 00 Postscheckamt Berlin West Konto-Nr 1175-101 Bankleitzahl 100 100 10

Die folgenden Beispiele erläutern die Anwendungsmöglichkeiten der erfindungsgemäßen Verbindungen die in Form der oben angegebenen Zubereitungen erfolgte.

BEISPIEL 15

Im Gewächshaus wurden die in der Tabelle aufgeführten erfindungsgemäßen Verbindungen in einer Aufwandmenge von 3,0kg Wirkstoff/ha emulgiert in 500 Liter Wasser/ha, auf Setaria und Ackerfuchsschwanz als Testpflanzen im Vor- und Nachauflaufverfahren gespritzt.

3 Wochen nach der Behandlung wurde das Behandlungsergebnis bonitiert, wobei

0 = keine Wirkung und

4 = Vernichtung der Pflanzen bedeutet.

Wie aus der Tabelle ersichtlich wird, wurde in der Regel eine Vernichtung der Testpflanzen erreicht.

| Erfindungsgemäße Verbindungen | Vorauflauf Setaria | Vorauflauf Ackerfuchsschwanz | Nachauflauf Setaria | Nachauflauf Ackerfuchsschwanz |
|---|---|---|---|---|
| 5-O-{2-/4-(6-Chlorbenzothiazol-2-yloxy)-phenoxy7-propionyl}-1,2:3,4-bis-O-isopropylidenxylit | 4 | 4 | 4 | 4 |
| 5-O-{2-/4-(Benzothiazol-2-yloxy)-phenoxy7-propionyl}-1,2:3,4-bis-O-isopropylidenxylit | 4 | 4 | 4 | 4 |
| 5-O-{2-/4-(6-Chlorbenzothiazol-2-yloxy)-phenoxy7-propionyl}-1,2:3,4-bis-O-(äthylpropyliden)-xylit | 4 | 4 | 4 | 4 |
| 5-O-{2-/4-(6-Chlorbenzothiazol-2-yloxy)-phenoxy7-propionyl}-1,2:3,4-bis-O-(1-methylpropyliden)-xylit | 4 | 4 | 4 | 4 |

-20-

| Erfindungsgemäße Verbindungen | Vorauflauf Setaria | Vorauflauf Ackerfuchs-schwanz | Nachauflauf Setaria | Nachauflauf Ackerfuhcs-schwanz |
|---|---|---|---|---|
| 5-O-{2-/4-(6-Chlorbenzothiazol-2-yloxy)-phenoxy7-propionyl}-1,2:3,4-bis-O-äthyliden-xylit | 4 | 4 | 4 | 4 |
| 5-O-{2-/4-(6-Chlorbenzoxazol-2-yloxy)-phenoxy7-propionyl}-1,2:3,4-bis-O-isopropyliden-xylit | 4 | 4 | 4 | 4 |
| 5-O-{2-/4-Benzoxazol-2-yloxy)-phenoxy7-propionyl}-1,2:3,4-bis-O-isopropylidenxylit | 4 | 4 | 4 | 4 |
| 5-O-{2-/4-(6-Chlorbenzothiazol-2-yloxy)-phenoxy7-propionyl}-1,2:3,4-bis-O-(methyl-octyl-methylen)-xylit | 4 | 4 | 4 | 4 |
| 5-O-{2-/4-(6-Chlorbenzothiazol-2-yloxy)-phenoxy7-propionyl}-1,2:3,4-bis-O-(4-chlorphenyl-methylen)-xylit | 4 | 4 | 4 | 4 |
| 5-O-{2-/4-(6-Chlorbenzoxazol-2-yloxy)-phenoxy7-propionyl}-1,2:3,4-bis-O-(äthylpropyliden)-xylit | 4 | 4 | 4 | 4 |
| 5-O-{2-/4-(6-Chlorbenzoxazol-2-yloxy)-phenoxy7-propionyl}-1,2:3,4-bis-O-(1-methylpropyl-iden)-xylit | 4 | 4 | 4 | 4 |
| 5-O-{2-/4-(6-chlorbenzoxazol-2-yloxy)-phenoxy7-propionyl}-1,2:3,4-bis-O-äthyliden-xylit | 4 | 4 | 4 | 4 |
| 5-O-{2-/4-(6-Chlorbenzoxazol-2-yloxy)-phenoxy7-propionyl}-1,2:3,4-bis-O-(methyl-octyl-methylen)-xylit | 4 | 4 | 4 | 4 |
| 5-O-{2-/4-(6-Chlorbenzoxazol-2-yloxy)-phenoxy7-propionyl}-1,2:3,4-bis-O-(4-chlorphenyl-methyl)-xylit | 4 | 4 | 4 | 4 |

-21-

Postanschrift: Schering Aktiengesellschaft, Postfach 65 03 11, D-1000 Berlin 65 · Fur Besucher Berlin-Wedding, Mullerstraße 170-178 Telegramme Scheringchemie Berlin

Vorstand Dr Herbert Asmis Dr Christian Bruhn Dr Heinz Hannse Horst Kramp Dr Klaus Pohle Dr Horst Witzel Vorsitzender des Aufsichtsrats Hans-Jurgen Hamann Sitz der Gesellschaft Berlin und Bergkamen Handelsregister AG Charlottenburg 93 HRB 283 und AG Kamen HRB 0061 Berliner Commerzbank AG Berlin, Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG, Konto-Nr 2415008 Bankleitzahl 100 700 00 Postscheckamt Berlin West Konto-Nr 1175-101 Bankleitzahl 100 100 10

## BEISPIEL 16

Im Gewächshaus wurden die aufgeführten Pflanzen nach dem Auflaufen mit der aufgeführten Verbindung in einer Aufwandmenge von 0,1 kg Wirkstoff/ha behandelt. Die Verbindungen wurden zu diesem Zweck als Emulsion mit 500 Liter Wasser/ha gleichmäßig über die Pflanzen versprüht. Hier zeigt 3 Wochen nach der Behandlung die erfindungsgemäße Verbindung eine hohe Selektivität bei ausgezeichneter Wirkung gegen das Unkraut.

14 Tage nach der Behandlung wurde der Behandlungserfolg nach dem Boniturschema 0 bis 10 bonitiert, wobei

0 = totale Vernichtung und

10 = keine Wirkung bedeuten.

| Erfindungsgemäße Verbindung | Echinochloa | Avena | Sorghum | Setaria | Bromus | Alopecurus | Digitaria | Zuckerrübe | Soja | Sonnenblume | Kartoffel | Raps | Baumwolle |
|---|---|---|---|---|---|---|---|---|---|---|---|---|---|
| 5-O-{2-[4-(6-Chlorbenzothiazol-2-yloxy)-phenoxy]-propionyl}-1,2:3,4-bis-O-isopropylidenxylit | 0 | 0 | 0 | 0 | 0 | 0 | 0 | 10 | 10 | 10 | 10 | 10 | 10 |

-22-

BEISPIEL 17

Im Gewächshaus wurden die aufgeführten Pflanzen nach dem Auflaufen mit den nachstehend angegebenen Komponenten oder deren Mischung behandelt und zu diesem Zweck als Emulsion mit 500 Liter Wasser/ha gleichmäßig über die Pflanzen versprüht.

3 Wochen nach der Behandlung wurde das Behandlungsergebnis bonitiert, wobei

0 = Vernichtung der Pflanzen und

10 = keine Schädigung bedeuten.

Wie aus der Tabelle ersichtlich wird, wirkte die erfindungsgemäße Mischung stark auf zahlreiche Problemunkräuter unter völliger Schonung der Zuckerrüben.

| Erfindungsgemäße Verbindung | Aufwandmenge kg/ha | Zuckerrübe | Avena | Sorghum | Cynodon dactylon | Digitaria | Setaria | Echinochloa | Alopecurus | Sinapis | Stellaria |
|---|---|---|---|---|---|---|---|---|---|---|---|
| 5-O-{2-(6-Chlorbenzothiazol-2-yloxy)-phenoxy]-propionyl}-1,2:3,4-bis-O-isopropylidenxylit (I) | 0,15 | 10 | 0 | 0 | 0 | 0 | 0 | 0 | 0 | 10 | 10 |
| (Phenmedipham) Methyl-N-(3-N'-3-methylphenyl-carbamoyloxyphenyl)-carbamat (II) | 0,7 | 10 | 5 | 8 | 10 | 8 | 8 | 7 | 6 | 0 | 0 |
| I+II | 0,7+0,15 | 10 | 0 | 0 | 0 | 0 | 0 | 0 | 0 | 0 | 0 |

-23-

# PATENTANSPRÜCHE

1. 2-Phenoxypropionsäurederivate von Pentiten vom Typ heterocyclischer Äther der allgemeinen Formel

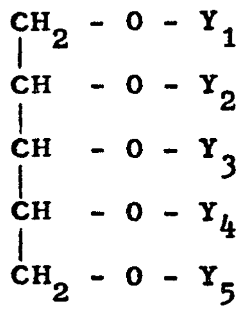

I

in der einer der Substituenten Y die Gruppe

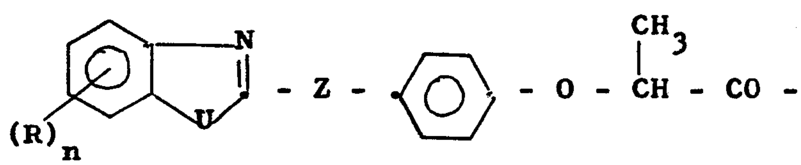

II

und die anderen vier Substituenten Y jeweils paarweise eine gegebenenfalls substituierte Methylengruppe der allgemeinen Formel

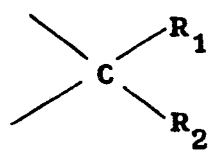

bedeuten, worin

$R_1$ und $R_2$ gleich oder verschieden sind und jeweils Wasserstoff, einen $C_1$-$C_{10}$-Alkylrest, einen ein- oder mehrfach durch Halogen, $C_1$-$C_6$-Alkoxy, Phenoxy und/oder Halogenphenoxy substituierten $C_1$-$C_{10}$-Alkylrest, einen Aryl-$C_1$-$C_3$-alkylrest, einen ein- oder mehrfach durch $C_1$-$C_6$-Alkyl, Halogen, $C_1$-$C_6$-Alkoxy, Nitro und/oder Trifluormethyl substituierten Aryl-$C_1$-$C_3$-alkylrest, einen $C_3$-$C_8$-cycloaliphatischen Kohlenwasserstoffrest, einen aromatischen Kohlenwasserstoffrest oder einen ein- oder mehrfach durch $C_1$-$C_6$-Alkyl, Halogen, $C_1$-$C_6$-Alkoxy, Nitro und/oder Trifluormethyl substituierten aromatischen Kohlenwasserstoff-

-24-

rest oder $R_1$ und $R_2$ gemeinsam mit dem angrenzenden C-Atom einen $C_3$-$C_8$-cycloaliphatischen Kohlenwasserstoffrest darstellen,
R Halogen-, $C_1$-$C_6$-Alkoxy, $C_1$-$C_6$-Alkyl, Nitro und/oder die Trifluormethylgruppe,
U und Z jeweils ein Sauerstoff- oder ein Schwefelatom und n die Zahlen 0, 1 oder 2 bedeuten.

2. 2-Phenoxypropionsäureester gemäß Anspruch 1, worin $R_1$ und $R_2$ gleich oder verschieden sind und jeweils Wasserstoff, Methyl, Äthyl, Propyl, Isopropyl, n-Butyl, sec.-Butyl, tert.-Butyl, 2,2-Dimethyl-1-propyl, n-Pentyl, n-Heptyl, n-Octyl, n-Decyl, Chlormethyl, Brommethyl, Fluormethyl, Dichlormethyl, Trifluormethyl, Trichlormethyl, Methoxymethyl, Äthoxymethyl, Phenoxymethyl, 4-Chlorphenoxymethyl, Chloräthyl, Bromäthyl, 2-Äthoxyäthyl, 2-Phenoxyäthyl, Cyclopropyl, Cyclopentyl, Cyclohexyl, Benzyl, 2-Phenyläthyl, Phenyl, 2-Chlorphenyl, 3-Chlorphenyl, 4-Chlorphenyl, 3,4-Dichlorphenyl, 4-Methoxyphenyl, 4-Nitrophenyl oder 2,4-Dichlorphenyl bedeuten oder
$R_1$ und $R_2$ gemeinsam unter Ausbildung einer Spiroverbindung eine Polymethylengruppe $-(CH_2)_m-$ darstellen, wobei m die Zahl 4 oder 5 darstellt.

3. 5-O-{2-/4-(6-Chlorbenzothiazol-2-yloxy)phenoxy7-propionyl}-1,2:3,4-bis-O-isopropylidenxylit

4. 5-O-{2-/4-(6-Chlorbenzoxazol-2-yloxy)-phenoxy7-propionyl}-1,2:3,4-bis-O-isopropylidenxylit

5. 5-O-{2-/4-(Benzothiazol-2-yloxy)-phenoxy7-propionyl}-1,2:3,4-bis-O-isopropylidenxylit

6. 5-O-{2-/4-(6-Chlorbenzothiazol-2-yloxy)-phenoxy7-propionyl}-1,2:3,4-bis-O-(äthylpropyliden)-xylit

7. 5-O-{2-/4-(6-Chlorbenzothiazol-2-yloxy)-phenoxy7-propionyl}-1,2:3,4-bis-O-(1-methylpropyliden)-xylit

-25-

Postanschrift: Schering Aktiengesellschaft, Postfach 65 03 11, D-1000 Berlin 65 · Fur Besucher Berlin-Wedding, Müllerstraße 170-178 Telegramme Scheringchemie Berlin.
Vorstand Dr. Herbert Asmis Dr. Christian Bruhn, Dr. Heinz Hannse, Horst Kramp, Dr. Klaus Pohle, Dr. Horst Witzel · Vorsitzender des Aufsichtsrats Hans-Jurgen Hamann Sitz der Gesellschaft Berlin und Bergkamen Handelsregister AG Charlottenburg 93 HRB 283 und AG Kamen HRB 0061 Berliner Commerzbank AG Berlin, Konto-Nr 108700600 Bankleitzahl 100 400 00 Berliner Handels- und Frankfurter Bank, Berlin Konto-Nr 70045224, Bankleitzahl 100 202 00 Deutsche Bank Berlin AG, Konto-Nr 2415008 Bankleitzahl 100 700 00 Postscheckamt Berlin West Konto-Nr 1175-101, Bankleitzahl 100 100 10

8. 5-O-{2-[4-(6-Chlorbenzothiazol-2-yloxy)-phenoxy]-propionyl}-1,2:3,4-bis-O-äthyliden-xylit

9. 5-O-{2-[4-(Benzoxazol-2-yloxy)-phenoxy]-propionyl}-1,2:3,4-bis-O-isopropylidenxylit

10. Verfahren zur Herstellung von 2-Phenoxypropionsäureestern gemäß Ansprüchen 1 bis 9, dadurch gekennzeichnet, daß man

A) Verbindungen der allgemeinen Formel

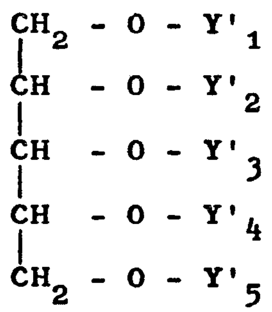

III

in der einer der Substituenten Y' Wasserstoff und die anderen vier Substituenten Y' jeweils paarweise eine gegebenenfalls substituierte Methylengruppe

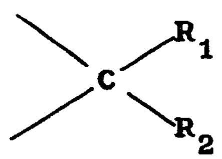

bedeuten mit Verbindungen der allgemeinen Formel

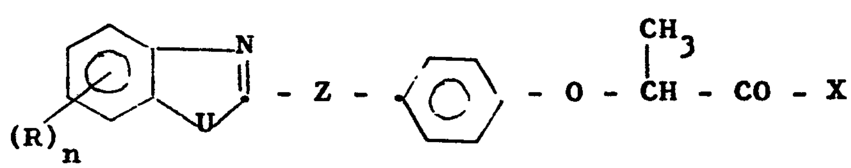

IV

gegebenenfalls in Gegenwart von säurebindenden Mitteln und/oder eines Katalysators reagieren läßt oder



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

B) Verbindungen der allgemeinen Formel

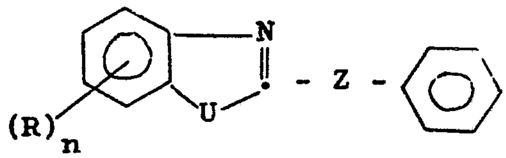

V

mit einem α-Halogenpropionsäurepentitester der allgemeinen Formel

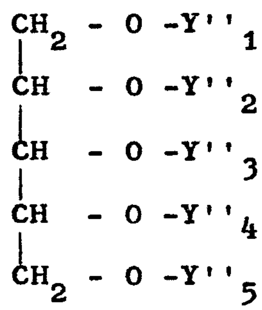

VI

in der einer der Substituenten Y'' die Gruppe

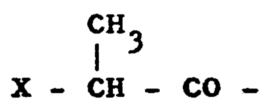

VII

und die anderen vier Substituenten Y'' jeweils paarweise eine gegebenenfalls substituierte Methylengruppe

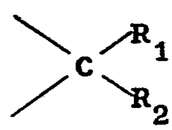

bedeuten, gegebenenfalls in Gegenwart von säurebindenden Mitteln und/oder eines Katalysators reagieren läßt oder, im Fall, daß Z allein Sauerstoff bedeutet,

C) Verbindungen der allgemeinen Formel

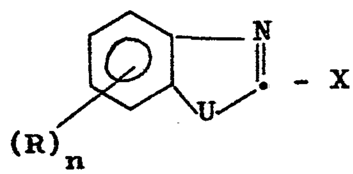

VIII

-27-

SCHERING
0141045

mit einem Hydrochinon - Milchsäureäther - derivat der allgemeinen Formel

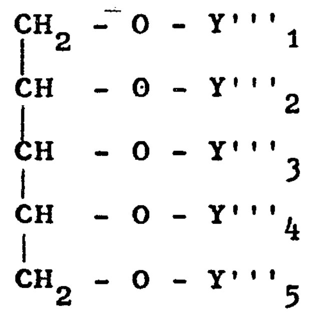

IX

in der eine der Substituenten Y''' die Gruppe

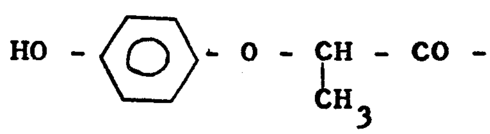

X

und die anderen vier Substituenten Y''' jeweils paarweise eine gegebenenfalls substituierte Methylengruppe der allgemeinen Formel

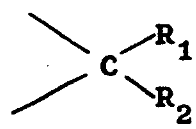

bedeuten, gegebenenfalls in Anwesenheit von säurebindenden Mitteln und/oder eines Katalysators reagieren läßt, worin R, $R_1$, $R_2$, n und Z die oben genannte Bedeutung haben und X ein Halogenatom, vorzugsweise Chlor oder Brom, darstellen.

11. Mittel mit herbizider Wirkung, gekennzeichnet durch einen Gehalt an mindestens einer Verbindung gemäß den Ansprüchen 1 bis 9.

12. Mittel mit herbizider Wirkung, gekennzeichnet durch einen Gehalt an mindestens einer Verbindung gemäß den Ansprüchen 1 bis 9 in Mischung mit mindestens einer Verbindung der allgemeinen Formel

-28-

Postanschrift: Schering Aktiengesellschaft, Postfach 65 03 11, D-1000 Berlin 65 · Fur Besucher Berlin-Wedding, Müllerstraße 170-178 · Telegramme: Scheringchemie Berlin
Vorstand: Dr Herbert Asmis Dr Christian Bruhn Dr Heinz Hannse. Horst Kramp. Dr. Klaus Pohle. Dr. Horst Witzel · Vorsitzender des Aufsichtsrats: Hans-Jürgen Hamann Sitz der Gesellschaft Berlin und Bergkamen Handelsregister AG Charlottenburg 93 HRB 283 und AG Kamen HRB 0061 · Berliner Commerzbank AG, Berlin, Konto-Nr 108700600 Bankleitzahl 100 400 00 Berliner Handels- und Frankfurter Bank. Berlin. Konto-Nr 70045224. Bankleitzahl 100 202 00 · Deutsche Bank Berlin AG. Konto-Nr 2415008. Bankleitzahl 100 700 00 Postscheckamt Berlin West Konto-Nr 11 75-101 Bankleitzahl 100 100 10

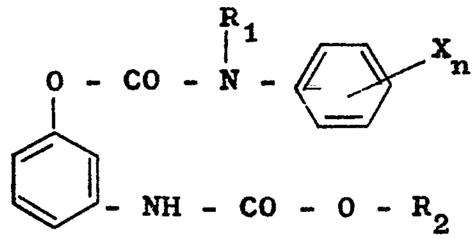

XI

in der

$R_1$ Wasserstoff oder einen $C_1$-$C_3$-Alkylrest, $R_2$ einen $C_1$-$C_3$-Alkylrest, X einen $C_1$-$C_3$-Alkylrest und/oder Halogen und/oder einen CN-Rest und n 0, 1, 2 oder 3 bedeuten, in einem Gewichtsverhältnis von 1:10 bis 10:1, vorzugsweise von 1:3 bis 3:1.

13. Mittel mit herbizider Wirkung gemäß Anspruch 12, gekennzeichnet durch einen Gehalt an 5-O-{2-[4-(6-Chlorbenzothiazol-2-yloxy)phenoxy]-propionyl}-1,2:3,4-bis-O-isopropylidenxylit oder 5-O-{2-[4-(Benzothiazol-2-yloxy)-phenoxy]-propionyl}-1,2:3,4-bis-O- isopropylidenxylit in Mischung mit
Methyl-N-(3-(N'-(3-methylphenyl)-carbamoyloxy)-phenyl)-carbamat,
Äthyl-N-(3-(N'-phenylcarbamoyloxy)-phenyl)-carbamat oder
Isopropyl-N-(3-(N'-äthyl-N'-phenylcarbamoyloxy)-phenyl)-carbamat.

14. Mittel mit herbizider Wirkung gemäß Anspruch 11 in Mischung mit Träger und/oder Hilfsstoffen.

15. Mittel mit herbizider Wirkung gemäß Anspruch 11 hergestellt nach Verfahren gemäß Anspruch 10.



Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600 Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008 Bankleitzahl 100 700 00 · Postscheckamt Berlin West, Konto-Nr. 1175-101, Bankleitzahl 100 100 10